# EUROPEAN PATENT SPECIFICATION

(11) **EP 1 531 843 B1**
(45) Date of publication and mention of the grant of the patent: **24.08.2011**
(21) Application number: 02805181.1
(22) Date of filing: 17.12.2002
(51) Int. Cl.: A61K 38/00, A61K 39/00, A61K 45/00, A61P 35/00, A61P 31/20

(54) **METHOD FOR TREATING CERVICAL CANCER**
VERFAHREN ZUR BEHANDLUNG VON GEBÄRMUTTERHALSKREBS
METHODE DE TRAITEMENT DU CANCER DU COL DE L'UTERUS

(30) Priority: 17.12.2001 US 341783 P
(43) Date of publication of application: 25.05.2005
(60) Divisional application: 08159216.4
(73) Proprietor: ZymoGenetics, Inc., Seattle, WA 98102 (US)
(72) Inventor: CHANDRASEKHER, Yasmin, A., Saratoga, CA 95070 (US); MCKERNAN, Patricia, A., Seattle, Washington 98112 (US)
(74) Representative: MacLean, Martin Robert
(86) International application number: PCT/US2002/040309
(87) International publication number: WO 2003/051384

(56) References cited:
- WO-A1-00/15264
- WO-A1-99/03982
- US-B1- 6 486 301
- BLUMBERG ET AL.: 'Interleukin 20: discovery, receptor identification and role in epidermal function' CELL vol. 104, no. 1, 12 January 2001, pages 9 - 19, XP000996379
- INTERNET CITATION, [Online] 1989, online Oxford English Dictionary Retrieved from the Internet: <URL:http://dictionary.oed.com/cgi/entry/50 045903?> [retrieved on 2010-03-12]

### SEQUENCE LISTING

<110> ZymoGenetics, Inc.
<120> METHOD FOR TREATING CERVICAL CANCER
<130> 01-44PC
<150> 60/341,783
   <151> 2001-12-17
<160> 43
<170> FastSEQ for Windows Version 3.0
<210> 1
   <211> 926
   <212> DNA
   <213> Homo sapiens
<220>
   <221> CDS
   <222> (45)...(572)
<400> 1
   ctttgaattc ctagctcctg tggtctccag atttcaggcc taag atg aaa gcc tct      56
   agt ctt gcc ttc agc ctt ctc tct gct gcg ttt tat ctc cta tgg act      104
   cct tcc act gga ctg aag aca ctc aat ttg gga agc tgt gtg atc gcc      152
   aca aac ctt cag gaa ata cga aat gga ttt tct gac ata cgg ggc agt      200
   gtg caa gcc aaa gat gga aac att gac atc aga atc tta agg agg act      248
   gag tct ttg caa gac aca aag cct gcg aat cga tgc tgc ctc ctg cgc      296
   cat ttg cta aga ctc tat ctg gac agg gta ttt aaa aac tac cag acc      344
   cct gac cat tat act ctc cgg aag atc agc agc ctc gcc aat tcc ttt      392
   ctt acc atc aag aag gac ctc cgg ctc tgt cat gcc cac atg aca tgc      440
   cat tgt ggg gag gaa gca atg aag aaa tac agc cag att ctg agt cac      488
   ttt gaa aag ctg gaa cct cag gca gca gtt gtg aag gct ttg ggg gaa      536
   cta gac att ctt ctg caa tgg atg gag gag aca gaa taggaggaaa           582
   gtgatgctgc tgctaagaat attcgaggtc aagagctcca gtcttcaata cctgcagagg    642
   aggcatgacc ccaaaccacc atctctttac tgtactagtc ttgtgctggt cacagtgtat    702
   cttatttatg cattacttgc ttccttgcat gattgtcttt atgcatcccc aatcttaatt    762
   gagaccatac ttgtataaga tttttgtaat atctttctgc tattggatat atttattagt    822
   taatatattt atttattttt tgctattaat gtatttaatt ttttacttgg gcatgaaact    882
   ttaaaaaaaa ttcacaagat tatatttata acctgactag agca                     926
<210> 2
   <211> 176
   <212> PRT
   <213> Homo sapiens
<400> 2
<210> 3
<211> 793
   <212> DNA
   <213> Homo sapiens
<220>
   <221> CDS
   <222> (45)...(497)
<400> 3
   ctttgaattc ctagctcctg tggtctccag atttcaggcc taag atg aaa gcc tct      56
                                                    Met Lys Ala Ser
                                                    1
   agt ctt gcc ttc agc ctt ctc tct gct gcg ttt tat ctc cta tgg act     104
   Ser Leu Ala Phe Ser Leu Leu Ser Ala Ala Phe Tyr Leu Leu Trp Thr
    5                  10                  15                  20
   cct tcc act gga ctg aag aca ctc aat ttg gga agc tgt gtg atc gcc     152
   Pro Ser Thr Gly Leu Lys Thr Leu Asn Leu Gly Ser Cys Val Ile Ala
                   25                  30                  35
   aca aac ctt cag gaa ata cga aat gga ttt tct gac ata cgg ggc agt     200
   Thr Asn Leu Gln Glu Ile Arg Asn Gly Phe Ser Asp Ile Arg Gly Ser
               40                  45                  50
   gtg caa gcc aaa gat gga aac att gac atc aga atc tta agg agg act     248
   Val Gln Ala Lys Asp Gly Asn Ile Asp Ile Arg Ile Leu Arg Arg Thr
           55                  60                  65
   gag tct ttg caa gac aca aag cct gcg aat cga tgc tgc ctc ctg cgc     296
   Glu Ser Leu Gln Asp Thr Lys Pro Ala Asn Arg Cys Cys Leu Leu Arg
       70                  75                  80
   cat ttg cta aga ctc tat ctg gac agg gta ttt aaa aac tac cag acc     344
   His Leu Leu Arg Leu Tyr Leu Asp Arg Val Phe Lys Asn Tyr Gln Thr
    85                  90                  95                 100
   cct gac cat tat act ctc cgg aag atc agc agc ctc gcc aat tcc ttt     392
   Pro Asp His Tyr Thr Leu Arg Lys Ile Ser Ser Leu Ala Asn Ser Phe
                   105                 110                 115
   ctt acc atc aag aag gac ctc cgg ctc tgt ctg gaa cct cag gca gca     440
   Leu Thr Ile Lys Lys Asp Leu Arg Leu Cys Leu Glu Pro Gln Ala Ala
               120                 125                 130
   gtt gtg aag gct ttg ggg gaa cta gac att ctt ctg caa tgg atg gag     488
   Val Val Lys Ala Leu Gly Glu Leu Asp Ile Leu Leu Gln Trp Met Glu
           135                 140                 145
   gag aca gaa taggaggaaa gtgatgctgc tgctaagaat attcgaggtc             537
   Glu Thr Glu
       150
   aagagctcca gtcttcaata cctgcagagg aggcatgacc ccaaaccacc atctctttac   597
   tgtactagtc ttgtgctggt cacagtgtat cttatttatg cattacttgc ttccttgcat   657
   gattgtcttt atgcatcccc aatcttaatt gagaccatac ttgtataaga tttttgtaat   717
   atctttctgc tattggatat atttattagt taatatattt atttattttt tgctattaat   777
   gtatttaatt ttttac                                                   793
<210> 4
   <211> 151
   <212> PRT
   <213> Homo sapiens
<400> 4
   Met Lys Ala Ser Ser Leu Ala Phe Ser Leu Leu Ser Ala Ala Phe Tyr
    1               5                  10                  15
   Leu Leu Trp Thr Pro Ser Thr Gly Leu Lys Thr Leu Asn Leu Gly Ser
               20                  25                  30
   Cys Val Ile Ala Thr Asn Leu Gln Glu Ile Arg Asn Gly Phe Ser Asp
           35                  40                  45
   Ile Arg Gly Ser Val Gln Ala Lys Asp Gly Asn Ile Asp Ile Arg Ile
       50                  55                  60
   Leu Arg Arg Thr Glu Ser Leu Gln Asp Thr Lys Pro Ala Asn Arg Cys
   65                  70                  75                  80
   Cys Leu Leu Arg His Leu Leu Arg Leu Tyr Leu Asp Arg Val Phe Lys
                   85                  90                  95
   Asn Tyr Gln Thr Pro Asp His Tyr Thr Leu Arg Lys Ile Ser Ser Leu
               100                 105                 110
   Ala Asn Ser Phe Leu Thr Ile Lys Lys Asp Leu Arg Leu Cys Leu Glu
           115                 120                 125
   Pro Gln Ala Ala Val Val Lys Ala Leu Gly Glu Leu Asp Ile Leu Leu
       130                 135                 140
   Gln Trp Met Glu Glu Thr Glu
   145                 150
<210> 5
   <211> 253
   <212> DNA
   <213> Homo sapiens
<400> 5
   ctttgaattc ctagctcctg tggtctccag atttcaggcc taagatgaaa gcctctagtc      60
   ttgccttcag ccttctctct gctgcgtttt atctcctatg gactccttcc actggactga     120
   agacactcaa tttgggaagc tgtgtgatcg ccacaaacct tcaggaaata cgaaatggat     180
   tttctgagat acggggcagt gtgcaagcca aagatggaaa cattgacatc agaatcttaa     240
   ggaggactga gtc                                                        253
<210> 6
   <211> 24
   <212> DNA
   <213> Homo sapiens
<400> 6
   attcctagct cctgtggtct ccag 24
<210> 7
   <211> 25
   <212> DNA
   <213> Homo sapiens
<400> 7
   ctctgctgcg ttttatctcc tatgg 25
<210> 8
   <211> 22
   <212> DNA
   <213> Homo sapiens
<400> 8
   tcccaaattg agtgtcttca gt 22
<210> 9
   <211> 45
   <212> DNA
   <213> Homo sapiens
<400> 9
   cacagcttcc acccttgagt gtcttcagtc cagtggaagg agtcc 45
<210> 10
   <211> 747
   <212> DNA
   <213> Homo sapiens
<400> 10
   ttttctgaca tacggggcag tgtgcaagcc aaagatggaa acattgacat cagaatctta      60
   aggaggactg agtctttgca agacacaaag cctgcgaatc gatgctgcct cctgcgccat     120
   ttgctaagac tctatctgga cagggtattt aaaaactacc agacccctga ccattatact     180
   ctccggaaga tcagcagcct cgccaattcc tttcttacca tcaagaagga cctccggctc     240
   tgtcatgccc acatgacatg ccattgtggg gaggaagcaa tgaagaaata cagccagatt     300
   ctgagtcact ttgaaaagct ggaacctcag gcagcagttg tgaaggcttt gggggaacta     360
   gacattcttc tgcaatggat ggaggagaca gaataggagg aaagtgatgc tgctgctaag     420
   aatattcgag gtcaagagct ccagtcttca atacctgcag aggaggcatg accccaaacc     480
   accatctctt tactgtacta gtcttgtgct ggtcacagtg tatcttattt atgcattact     540
   tgcttccttg catgattgtc tttatgcatc cccaatctta attgagacca tacttgtata     600
   agatttttgt aatatctttc tgctattgga tatatttatt agttaatata tttatttatt     660
   ttttgctatt aatgtattta attttttact tgggcatgaa actttaaaaa aaattcacaa     720
   gattatattt ataacctgac tagagca                                         747
<210> 11
   <211> 614
   <212> DNA
   <213> Homo sapiens
<400> 11
   ttttctgaca tacggggcag tgtgcaagcc aaagatggaa acattgacat cagaatctta      60
   aggaggactg agtctttgca agacacaaag cctgcgaatc gatgctgcct cctgcgccat     120
   ttgctaagac tctatctgga cagggtattt aaaaactacc agacccctga ccattatact     180
   ctccggaaga tcagcagcct cgccaattcc tttcttacca tcaagaagga cctccggctc     240
   tgtctggaac ctcaggcagc agttgtgaag gctttggggg aactagacat tcttctgcaa     300
   tggatggagg agacagaata ggaggaaagt gatgctgctg ctaagaatat tcgaggtcaa     360
   gagctccagt cttcaatacc tgcagaggag gcatgacccc aaaccaccat ctctttactg     420
   tactagtctt gtgctggtca cagtgtatct tatttatgca ttacttgctt ccttgcatga     480
   ttgtctttat gcatccccaa tcttaattga gaccatactt gtataagatt tttgtaatat     540
   ctttctgcta ttggatatat ttattagtta atatatttat ttattttttg ctattaatgt     600
   atttaatttt ttac                                                       614
<210> 12
   <211> 152
   <212> PRT
   <213> Homo sapiens
<400> 12
   Leu Lys Thr Leu Asn Leu Gly Ser Cys Val Ile Ala Thr Asn Leu Gln
   1 5 10 15
   Glu Ile Arg Asn Gly Phe Ser Asp Ile Arg Gly Ser Val Gln Ala Lys
   20 25 30
   Asp Gly Asn Ile Asp Ile Arg Ile Leu Arg Arg Thr Glu Ser Leu Gln
   35 40 45
   Asp Thr Lys Pro Ala Asn Arg Cys Cys Leu Leu Arg His Leu Leu Arg
   50 55 60
   Leu Tyr Leu Asp Arg Val Phe Lys Asn Tyr Gln Thr Pro Asp His Tyr
   65 70 75 80
   Thr Leu Arg Lys Ile Ser Ser Leu Ala Asn Ser Phe Leu Thr Ile Lys
   85 90 95
   Lys Asp Leu Arg Leu Cys His Ala His Met Thr Cys His Cys Gly Glu
   100 105 110
   Glu Ala Met Lys Lys Tyr Ser Gln Ile Leu Ser His Phe Glu Lys Leu
   115 120 125
   Glu Pro Gln Ala Ala Val Val Lys Ala Leu Gly Glu Leu Asp Ile Leu
   130 135 140
   Leu Gln Trp Met Glu Glu Thr Glu
   145 150
<210> 13
   <211> 127
   <212> PRT
   <213> Homo sapiens
<400> 13
   Leu Lys Thr Leu Asn Leu Gly Ser Cys Val Ile Ala Thr Asn Leu Gln
   1 5 10 15
   Glu Ile Arg Asn Gly Phe Ser Asp Ile Arg Gly Ser Val Gln Ala Lys
   20 25 30
   Asp Gly Asn Ile Asp Ile Arg Ile Leu Arg Arg Thr Glu Ser Leu Gln
   35 40 45
   Asp Thr Lys Pro Ala Asn Arg Cys Cys Leu Leu Arg His Leu Leu Arg
   50 55 60
   Leu Tyr Leu Asp Arg Val Phe Lys Asn Tyr Gln Thr Pro Asp His Tyr
   65 70 75 80
   Thr Leu Arg Lys Ile Ser Ser Leu Ala Asn Ser Phe Leu Thr Ile Lys
   85 90 95
   Lys Asp Leu Arg Leu Cys Leu Glu Pro Gln Ala Ala Val Val Lys Ala
   100 105 110
   Leu Gly Glu Leu Asp Ile Leu Leu Gln Trp Met Glu Glu Thr Glu
   115 120 125
<210> 14
   <211> 15
   <212> PRT
   <213> Homo sapiens
<400> 14
   Ile Ala Thr Asn Leu Gln Glu Ile Arg Asn Gly Phe Ser Asp Ile
   1 5 10 15
<210> 15
   <211> 15
   <212> PRT
   <213> Homo sapiens
<400> 15
   Leu Asp Arg Val Phe Lys Asn Tyr Gln Thr Pro Asp His Tyr Thr
    1               5                  10                  15
<210> 16
   <211> 15
   <212> PRT
   <213> Homo sapiens
<400> 16
   Leu Ala Asn Ser Phe Leu Thr Ile Lys Lys Asp Leu Arg Leu Cys
    1               5                  10                  15
<210> 17
   <211> 15
   <212> PRT
   <213> Homo sapiens
<400> 17 <210> 18
   <211> 824
   <212> DNA
   <213> Mus musculus
<220>
   <221> CDS
   <222> (71)...(598)
<400> 18
<210> 19
   <211> 176
   <212> PRT
   <213> Mus musculus
<400> 19
   Met Lys Gly Phe Gly Leu Ala Phe Gly Leu Phe Ser Ala Val Gly Phe
    1               5                  10                  15
   Leu Leu Trp Thr Pro Leu Thr Gly Leu Lys Thr Leu His Leu Gly Ser
               20                  25                  30
   Cys Val Ile Thr Ala Asn Leu Gln Ala Ile Gln Lys Glu Phe Ser Glu
           35                  40                  45
   Ile Arg Asp Ser Val Gln Ala Glu Asp Thr Asn Ile Asp Ile Arg Ile
       50                  55                  60
   Leu Arg Thr Thr Glu Ser Leu Lys Asp Ile Lys Ser Leu Asp Arg Cys
   65                  70                  75                  80
   Cys Phe Leu Arg His Leu Val Arg Phe Tyr Leu Asp Arg Val Phe Lys
                   85                  90                  95
   Val Tyr Gln Thr Pro Asp His His Thr Leu Arg Lys Ile Ser Ser Leu
               100                 105                 110
   Ala Asn Ser Phe Leu Ile Ile Lys Lys Asp Leu Ser Val Cys His Ser
           115                 120                 125
   His Met Ala Cys His Cys Gly Glu Glu Ala Met Glu Lys Tyr Asn Gln
       130                 135                 140
   Ile Leu Ser His Phe Ile Glu Leu Glu Leu Gln Ala Ala Val Val Lys
   145                 150                 155                 160
   Ala Leu Gly Glu Leu Gly Ile Leu Leu Arg Trp Met Glu Glu Met Leu
                   165                 170                 175
<210> 20
   <211> 152
   <212> PRT
   <213> Mus musculus
<400> 20
   Leu Lys Thr Leu His Leu Gly Ser Cys Val Ile Thr Ala Asn Leu Gln
    1               5                  10                  15
   Ala Ile Gln Lys Glu Phe Ser Glu Ile Arg Asp Ser Val Gln Ala Glu
               20                  25                  30
   Asp Thr Asn Ile Asp Ile Arg Ile Leu Arg Thr Thr Glu Ser Leu Lys
           35                  40                  45
   Asp Ile Lys Ser Leu Asp Arg Cys Cys Phe Leu Arg His Leu Val Arg
       50                  55                  60
   Phe Tyr Leu Asp Arg Val Phe Lys Val Tyr Gln Thr Pro Asp His His
   65                  70                  75                  80
   Thr Leu Arg Lys Ile Ser Ser Leu Ala Asn Ser Phe Leu Ile Ile Lys
                   85                  90                  95
   Lys Asp Leu Ser Val Cys His Ser His Met Ala Cys His Cys Gly Glu
               100                 105                 110
   Glu Ala Met Glu Lys Tyr Asn Gln Ile Leu Ser His Phe Ile Glu Leu
           115                 120                 125
   Glu Leu Gln Ala Ala Val Val Lys Ala Leu Gly Glu Leu Gly Ile Leu
       130                 135                 140
   Leu Arg Trp Met Glu Glu Met Leu
   145                 150
<210> 21
   <211> 16
   <212> PRT
   <213> Mus musculus
<400> 21
   Ile Thr Ala Asn Leu Gln Ala Ile Gln Lys Glu Phe Ser Glu Ile Arg
   1 5 10 15
<210> 22
   <211> 15
   <212> PRT
   <213> Mus musculus
<400> 22
   Leu Asp Arg Val Phe Lys Val Tyr Gln Thr Pro Asp His His Thr
   1 5 10 15
<210> 23
   <211> 15
   <212> PRT
   <213> Mus musculus
<400> 23
   Leu Ala Asn Ser Phe Leu Ile Ile Lys Lys Asp Leu Ser Val Cys
   1 5 10 15
<210> 24
   <211> 15
   <212> PRT
   <213> Mus muculus
<400> 24
   Val Val Lys Ala Leu Gly Glu Leu Gly Ile Leu Leu Arg Trp Met
   1 5 10 15
<210> 25
   <211> 144
   <212> PRT
   <213> Mus muculus
<400> 25
   Cys Val Ile Thr Ala Asn Leu Gln Ala Ile Gln Lys Glu Phe Ser Glu
    1               5                  10                  15
   Ile Arg Asp Ser Val Gln Ala Glu Asp Thr Asn Ile Asp Ile Arg Ile
               20                  25                  30
   Leu Arg Thr Thr Glu Ser Leu Lys Asp Ile Lys Ser Leu Asp Arg Cys
           35                  40                  45
   Cys Phe Leu Arg His Leu Val Arg Phe Tyr Leu Asp Arg Val Phe Lys
       50                  55                  60
   Val Tyr Gln Thr Pro Asp His His Thr Leu Arg Lys Ile Ser Ser Leu
   65                  70                  75                  80
   Ala Asn Ser Phe Leu Ile Ile Lys Lys Asp Leu Ser Val Cys His Ser
                   85                  90                  95
   His Met Ala Cys His Cys Gly Glu Glu Ala Met Glu Lys Tyr Asn Gln
               100                 105                 110
   Ile Leu Ser His Phe Ile Glu Leu Glu Leu Gln Ala Ala Val Val Lys
           115                 120                 125
   Ala Leu Gly Glu Leu Gly Ile Leu Leu Arg Trp Met Glu Glu Met Leu
       130                 135                 140
<210> 26
   <211> 144
   <212> PRT
   <213> Homo sapiens
<400> 26
   Cys Val Ile Ala Thr Asn Leu Gln Glu Ile Arg Asn Gly Phe Ser Asp
    1               5                  10                  15
   Ile Arg Gly Ser Val Gln Ala Lys Asp Gly Asn Ile Asp Ile Arg Ile
               20                  25                  30
   Leu Arg Arg Thr Glu Ser Leu Gln Asp Thr Lys Pro Ala Asn Arg Cys
           35                  40                  45
   Cys Leu Leu Arg His Leu Leu Arg Leu Tyr Leu Asp Arg Val Phe Lys
       50                  55                  60
   Asn Tyr Gln Thr Pro Asp His Tyr Thr Leu Arg Lys Ile Ser Ser Leu
   65                  70                  75                  80
   Ala Asn Ser Phe Leu Thr Ile Lys Lys Asp Leu Arg Leu Cys His Ala
                   85                  90                  95
   His Met Thr Cys His Cys Gly Glu Glu Ala Met Lys Lys Tyr Ser Gln
               100                 105                 110
   Ile Leu Ser His Phe Glu Lys Leu Glu Pro Gln Ala Ala Val Val Lys
           115                 120                 125
   Ala Leu Gly Glu Leu Asp Ile Leu Leu Gln Trp Met Glu Glu Thr Glu
       130                 135                 140
<210> 27
   <211> 38
   <212> PRT
   <213> Homo sapiens
<400> 27
   Cys Gly Glu Glu Ala Met Lys Lys Tyr Ser Gln Ile Leu Ser His Phe
   1 5 10 15
   Glu Lys Leu Glu Pro Gln Ala Ala Val Val Lys Ala Leu Gly Glu Leu
   20 25 30
   Asp Ile Leu Leu Gln Trp
   35
<210> 28
   <211> 71
   <212> PRT
   <213> Homo sapiens
<400> 28
   Ile Ala Thr Asn Leu Gln Glu Ile Arg Asn Gly Phe Ser Asp Ile Arg
   1 5 10 15
   Gly Ser Val Gln Ala Lys Asp Gly Asn Ile Asp Ile Arg Ile Leu Arg
   20 25 30
   Arg Thr Glu Ser Leu Gln Asp Thr Lys Pro Ala Asn Arg Cys Cys Leu
   35 40 45
   Leu Arg His Leu Leu Arg Leu Tyr Leu Asp Arg Val Phe Lys Asn Tyr
   50 55 60
   Gln Thr Pro Asp His Tyr Thr
   65 70
<210> 29
   <211> 92
   <212> PRT
   <213> Homo sapiens
<400> 29
   Ile Ala Thr Asn Leu Gln Glu Ile Arg Asn Gly Phe Ser Asp Ile Arg
   1 5 10 15
   Gly Ser Val Gln Ala Lys Asp Gly Asn Ile Asp Ile Arg Ile Leu Arg
   20 25 30
   Arg Thr Glu Ser Leu Gln Asp Thr Lys Pro Ala Asn Arg Cys Cys Leu
   35 40 45
   Leu Arg His Leu Leu Arg Leu Tyr Leu Asp Arg Val Phe Lys Asn Tyr
   50 55 60
   Gln Thr Pro Asp His Tyr Thr Leu Arg Lys Ile Ser Ser Leu Ala Asn
   65 70 75 80
   Ser Phe Leu Thr Ile Lys Lys Asp Leu Arg Leu Cys
   85 90
<210> 30
   <211> 82
   <212> PRT
   <213> Homo sapiens
<210> 31
   <211> 36
   <212> PRT
   <213> Homo sapiens
<400> 31
   Leu Asp Arg Val Phe Lys Asn Tyr Gln Thr Pro Asp His Tyr Thr Leu
    1               5                  10                  15
   Arg Lys Ile Ser Ser Leu Ala Asn Ser Phe Leu Thr Ile Lys Lys Asp
               20                  25                  30
   Leu Arg Leu Cys
           35
<210> 32
   <211> 61
   <212> PRT
   <213> Homo sapiens
<400> 32 <210> 33
   <211> 756
   <212> DNA
   <213> Mus musculus
<220>
   <221> CDS
   <222> (71)...(532)
<400> 33
<210> 34
   <211> 154
   <212> PRT
   <213> Mus musculus
<400> 34
   Met Lys Gly Phe Gly Leu Ala Phe Gly Leu Phe Ser Ala Val Gly Phe
   1               5                   10                  15
   Leu Leu Trp Thr Pro Leu Thr Gly Leu Lys Thr Leu His Leu Gly Ser
               20                  25                  30
   Cys Val Ile Thr Ala Asn Leu Gln Ala Ile Gln Lys Glu Phe Ser Glu
           35                  40                  45
   Ile Arg Asp Ser Val Ser Leu Asp Arg Cys Cys Phe Leu Arg His Leu
       50                  55                  60
   Val Arg Phe Tyr Leu Asp Arg Val Phe Lys Val Tyr Gln Thr Pro Asp
   65                  70                  75                  80
   His His Thr Leu Arg Lys Ile Ser Ser Leu Ala Asn Ser Phe Leu Ile
                   85                  90                  95
   Ile Lys Lys Asp Leu Ser Val Cys His Ser His Met Ala Cys His Cys
               100                 105                 110
   Gly Glu Glu Ala Met Glu Lys Tyr Asn Gln Ile Leu Ser His Phe Ile
           115                 120                 125
   Glu Leu Glu Leu Gln Ala Ala Val Val Lys Ala Leu Gly Glu Leu Gly
       130                 135                 140
   Ile Leu Leu Arg Trp Met Glu Glu Met Leu
   145                 150
<210> 35
   <211> 130
   <212> PRT
   <213> Mus musculus
<400> 35
   Leu Lys Thr Leu His Leu Gly Ser Cys Val Ile Thr Ala Asn Leu Gln
   1               5                   10                  15
   Ala Ile Gln Lys Glu Phe Ser Glu Ile Arg Asp Ser Val Ser Leu Asp
               20                  25                  30
   Arg Cys Cys Phe Leu Arg His Leu Val Arg Phe Tyr Leu Asp Arg Val
           35                  40                  45
   Phe Lys Val Tyr Gln Thr Pro Asp His His Thr Leu Arg Lys Ile Ser
       50                  55                  60
   Ser Leu Ala Asn Ser Phe Leu Ile Ile Lys Lys Asp Leu Ser Val Cys
   65                  70                  75                  80
   His Ser His Met Ala Cys His Cys Gly Glu Glu Ala Met Glu Lys Tyr
                   85                  90                  95
   Asn Gln Ile Leu Ser His Phe Ile Glu Leu Glu Leu Gln Ala Ala Val
               100                 105                 110
   Val Lys Ala Leu Gly Glu Leu Gly Ile Leu Leu Arg Trp Met Glu Glu
           115                 120                 125
   Met Leu
       130
<210> 36
   <211> 27
   <212> DNA
   <213> Homo sapiens
<400> 36
   agattctatc tggacaggt attcaaa 27
<210> 37
   <211> 17
   <212> DNA
   <213> Homo sapiens
<400> 37
   gcgaggctga tctttct 17
<210> 38
   <211> 25
   <212> DNA
   <213> Mus musculis
   <400> 38
   tggcgaggct gctgatcttt ctcag 25
<210> 39
   <211> 25
   <212> DNA
   <213> Mus musculis
<400> 39
   ctttatgtct ttcaaagact cagtc 25 <210> 40
   <211> 26
   <212> DNA
   <213> Mus musculis
<400> 40
   catcagaatt ttaaggacga ctgagt 26
<210> 41
   <211> 25
   <212> DNA
   <213> Mus musculis
<400> 41
   ggtggtcagg ggtctggtag acttt 25
<210> 42
   <211> 23
   <212> DNA
   <213> Mus musculis
<400> 42
   ggtgcatatt cctggtggct aga 23
<210> 43
<211> 25
   <212> DNA
   <213> Mus musculis
<400> 43

## Description

According to the American Cancer Society, 12,800 new cases of invasive cervical cancer would be diagnosed in the United States in 1999. During the same year, 4800 patients were expected to die of the disease. This represents approximately 1.8% of all cancer deaths in women and 18% of gynecological cancer deaths. However, for women aged 20 to 39 years of age, cervical cancer is the second leading cause of cancer deaths. Molecular and epidemiologic studies have demonstrated a strong relationship between human papillomavirus (HPV), cervical intraepithelial neoplasia, (CIN), and invasive carcinoma of the cervix. Thus, there is a need to develop new therapeutic entities for the treatment of human papillomavirus infection, cervical intraepithelial neoplasia and carcinoma of the cervix.

WO 00/15264 describes *in situ* injection of antigen-presenting cells with genetically enhanced cytokine expression.

WO 99/03982 describes interleukin-20.

WO 99/27103 describes mammalian cytokine-like polypeptide-10.

The present invention provides the use of IL-20 for the manufacture of a medicament for the treatment of cervical cancer, as defined in the claims.

The present invention also provides IL-20 for use in treating cervical cancer, as defined in the claims.

The present invention further provides an *in vitro* method for inhibiting the growth and/or proliferation of cervical cancer cells, comprising contacting the cells with IL-20, as defined in the claims.

Interteukin-20 (formally called Zcyto10) can be produced according to the method described in WO99/27103. The human IL-20 petypeptide is comprised of a sequence of 176 amino acids with the initial Met as shown in SEQ ID NO:1 and SEQ ID NO: 2. It is belleved that amino residues 1-24 are signal sequence, and the mature IL-20 polypeptide is represented by the amino acid sequence comprised of residue 25, a leucine, through amino acid residue 176, a glutamic acid residue, also defined by SEQ ID NO:12. The application describes the sequences of SEQ ID NO: 3 and SEQ ID NO: 4. The polypeptide of SEQ ID NO: 4 is comprised of 151 amino acid residues wherein amino acids 1-24 comprise a signal sequence and the mature sequence is comprised of amino acid residue 25, a leucine, through amino acid 151 a glutamic acid, also defined by SEQ ID NO:13. Another active variant is comprised of amino acid residue 33, a cysteine, through amino acid residue 176 of SEQ ID NO:2. This variant is also defined by SEQ ID NO:26.

Mouse IL-20 is also a polypeptide composed of 176 amino acid residues as defined by SEQ ID NOs:18 and 19. Mouse IL-20 has a signal sequence extending from amino acid residue 1, a methionine, extending to and including amino acid residue 24, a glycine of SEQ m NO:19. Thus, the mature mouse IL-20 extends from amino acid residue 25, a leucine, to and including amino acid residues 176 a leucine of SEQ ID NO:19, also defined by SEQ ID NO:20. Another active variant is believed to extend from amino acid 33, a cysteine, through amino acid 176, of SEQ ID NO:19. This variant is also defined by SEQ ID NO:25.

A variant of mouse IL-20 is defined by SEQ ID NOs: 33 and 34. This variant is 154 amino acid residues in length and has a signal sequence extending from amino acid residue 1, a methionine, to and including amino acid residue 24, a glycine, of SEQ ID NO:34. Thus, the mature sequence extends from amino acid residue 25, a leucine, to and including amino acid residue 154, a leucine, of SEQ ID NO:34. The mature sequence is also defined by SEQ ID NO:35.

### Pathology of Cervical Cancer

Cervical dysplasia cells and cervical intraepithelial neoplasia (CIN) cells develop into invasive cervical cancer over a number of years. CIN grades I, II and m correspond to mild, moderate, and severe cervical dysplasia. CIN III, which includes severe dysplasia and carcinoma *in situ,* is unlikely to regress spontaneously and, if untreated, may eventually penetrate the basement membrane, becoming invasive carcinoma. Squamous cell carcinoma accounts for 80 to 85% of all cervical cancers; adenocarcinomas account for most of the rest. Invasive cervical cancer usually spreads by direct extension into surrounding tissues and the vagina or via the lymphatics to the pelvic and para-aortic lymph nodes drained by the cervix. Hematologic spread is possible.

### Symptoms, Signs and Diagnosis of Cervical Cancer

CIN is usually asymptomatic and discovered because of an abnormal Pap smear. Patients with early-stage cervical cancer usually present with irregular vaginal bleeding, which is most often postcoital, but intermenstrual bleeding or menometrorrhagia may occur. Patients with larger cervical cancers or advanced-stage disease may present with foul-smelling vaginal discharge, abnormal vaginal bleeding, or pelvic pain. Obstructive uropathy, back pain, and leg swelling are manifestations of late-stage disease. Suspicious lesions, generally first detected by a Pap smear are biopsied. If clinical disease is invasive, staging is performer on the basis of the physical examination, with a metastatic survey including cystoscopy, sigmoidoscopy, IV pyelography, chest x-ray, and skeletal x-rays.

### Treatment of Cervical Cancer with IL-20

As defined in the claims, IL-2O can be used for the manufature of a medicament for treating cervical cancer in a female mammal, particularly a human female, afflicted with the disease. IL-20 can be for administration intralesionally, or intramuscularly for localized disease. For metastatic disease, IL-20 can also be for administration intraperitoneal administration including intravenous administration. IL-20 can be for administration alone or in conjunction with standard therapies such as surgery, radiation or other chemotherapeutic agents such as bleomycin, chlorambucil, epirubicin, 5-fluorouracil, ifosfamide, mitomycin, methotrexate, vincristine, cisplatin and vinblastine.

### Symptoms Signs and Diagnosis

Genital warts usually appear as soft, moist, minute pink or gray polyps that enlarge, may become pedunculated, and am usually found in clusters. The surfaces resemble the surface of cauliflower. In men they occur most commonly on warm, moist surfaces in the subpreputial area, on the coronal sulcus, within the urethral meatus, and on the penile shaft. In women, the vulva, the vaginal wall, the cervix, and the perineum may become involved They are particularly common in the perianal region and rectum in homosexual men. Growth rates vary, but pregnancy, immunosuppression, or maceration of the skin may accelerate both the growth of individual lesions and their spread. Genital warts usually can be identified by their appearance but must be differentiated from the flat-topped condyloma lata of secondary syphilis. Biopsies of atypical or persistent warts may be necessary to exclude carcinoma.

IL-20 can be administered directly into lesions containing cells infected with HPV alone or with standard therapies such as intaferon alpha or interferon beta both of which are commercially available, Interferon alpha is available from Schering Corporation of Kenilworth, New Jersey and is called INTRON A®. Interferon beta is produced by Biogen of Cambridge, MA and is called AVONEX ®. IL-20 can also be administered with other standard therapies for traeting HPV including antimitotics such as podaphyllotoxin, padophyllin, or 5-fluorouracil; caustics such as trichloroacetic acid; or interferon inducers such as imiquimod:

The quantities of IL-20 for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include, intravenous, peritoneal, intramuscular, or intralesional. Pharmaceutically acceptable carriers will include water, saline, buffers to name just a few. Dosage ranges would ordinarily be expected from 1µg to 1000µg per kilogram of body weight per day. However, the doses may be higher or lower as can be determined by a medical doctor with ordinary skill in the art. Excipients and stabilizers can possible be added. These include glycine, histidine, glutamate, aspartate, sugars, sucrose, trehalose, galactose sorbitol, arginime, D-and/or L-amino acids, sugar alcohols, lactose, maltose, threonine, lysine, methionine, isoleucine, a surface active agent such as TWEEN 80, TWEEN 20, polyethylene glycol (PEG) (particularly those PEGs having molecular weights between 1000 and 35000 Da), cetyl alcohol, polyvinylpyrrolidone, polyvinyl alcohol, lanolin alcohol and sorbitan. A reducing agent may be included, such as cysteine, N-acetylcysteine, and thioglycerol. For a complete discussion of drug formulations and dosage ranges *see* Remington's Pharmaceutical Sciences, 18th Ed., (Mack Publishing Co., Easton, Penn., 1996), and Goodman and Gilman's: The Pharmacological Bases of Therapeutics,9th Ed. (Pergamon Press 1996).

IL-20 can also used be used for administration in conjunction with other treatments for cervical cancer such as radiation and chemotherapy. Examples of chemotherapeutic agents include bleomycin, chlorambucil, epirubicin, 5-fluorouracil, ifosfamide, mitomycin, methotrexate, vincristine, cisplatin and vinblastine.

### Example

We tested IL-20 in a HeLa299 cytotoxicity assay to measure the ability of IL-20 to prevent cells from growing during normal growth conditions. We used MTT reagent (Promega, Madison, USA) as our detection and readout for this cell inhibition assay. Procedure of a cytoxicity assay:
Day 1- Plate cells out in complete growth media (with serum) at 5000cells/well in a 96well format and let them incubate overnight at 37degrees and 5% CO2.
Day 2- Dump off media and add a dose response of appropriate ligands in complete growth media (IL-20, zmdal, and MDA7 at 10, 100, and 1000 ng/ml.), along with a positive control retinoic acid (100uM) in complete growth media, while leaving some wells in complete growth media as controls of how the cells normally grow under normal conditions. Put the cells in incubator and let the assay go for 72hrs.
Day 5- Add 15ul/well of MTT reagent, let cells inc. for 4hrs., then add 100ul of stop solution, let cells inc. for an additional 1hr., then read the plate on a multilabel counter ( Victor2, PerkinElmer Life Sciences Inc., Boston). The MTT protocol will give you two readings, one at a 650 wavelength (background) and one at a 572 wavelength. Subtract the 650 reading from the 572 reading to get your actual output. These numbers are averaged and converted to a % inhibition value.

Results:
- Retnoic Acid gave a 53% inhibition of growth (positive control)
- IL-20 gave a maximal 20% inhibition of growth

## Claims

1. Use of a polypeptide selected from the group consisting of:
(a) a polypeptide consisting of amino acid residues 33-176 of SEQ ID NO: 2;
(b) a polypeptide consisting of amino acid residues 25-176 of SEQ ID NO: 2;
(c) a polypeptide consisting of amino acid residues 1-176 of SEQ ID NO: 2;
(d) a polypeptide consisting of amino acid residues 25-151 of SEQ ID NO: 4;and
(e) a polypeptide consisting of amino acid residues 1-151 of SEQ ID NO: 4;
for the manufacture of a medicament for the treatment of cervical cancer.

2. Use according to Claim 1 for inhibiting the growth and/ or proliferation of cervical cancer cells.

3. Use according to Claim 2, wherein the medicament is for administration in conjunction with treatment of the cells with radiation.

4. Use according to Claim 2, wherein the medicament is for administration in conjunction with treatment of the cells with one or more additional chemotherapeutic agents.

5. Use according to Claim 4, wherein the chemotherapeutic agent is selected from the group consisting of bleomycin, chlorambucil, epirubicin, 5-fluorouracil, ifosfamide, mitomycin, methotrexate, vincristine, cisplatin, and vinblastine.

6. A polypeptide selected from the group consisting of:
(a) a polypeptide consisting of amino acid residues 33-176 of SEQ ID NO: 2;
(b) a polypeptide consisting of amino acid residues 25-176 of SEQ ID NO: 2;
(c) a polypeptide consisting of amino acid residues 1-176 of SEQ ID NO: 2;
(d) a polypeptide consisting of amino acid residues 25-151 of SEQ ID NO: 4; and
(e) a polypeptide consisting of amino acid residues 1-151 of SEQ ID NO: 4;
for use in the treatment of cervical cancer.

7. The polypeptide for use according to Claim 6, for inhibiting the growth and/ or proliferation of cervical cancer cells.

8. The polypeptide for use according to Claim 7, for administration in conjunction with treatment of the cells with radiation.

9. The polypeptide for use according to Claim 7, for administration in conjunction with treatment of the cells with one or more additional chemotherapeutic agents.

10. The polypeptide for use according to Claim 9, wherein the chemotherapeutic agent is selected from the group consisting of bleomycin, chlorambucil, epirubicin, 5-fluorouracil, ifosfamide, mitomycin, methotrexate, vincristine, cisplatin, and vinblastine.

11. An *in vitro* method for inhibiting the growth and/ or proliferation of cervical cancer cells, comprising contacting the cells with a polypeptide selected from the group consisting of:
(a) a polypeptide consisting of amino acid residues 33-176 of SEQ ID NO: 2;
(b) a polypeptide consisting of amino acid residues 25-176 of SEQ ID NO: 2;
(c) a polypeptide consisting of amino acid residues 1-176 of SEQ ID NO: 2;
(d) a polypeptide consisting of amino acid residues 25-151 of SEQ ID NO: 4; and
(e) a polypeptide consisting of amino acid residues 1-151 of SEQ ID NO: 4.

12. The *in vitro* method according to Claim 11, wherein the cervical cancer cells are treated with radiation in conjunction with said polypeptide.

13. The *in vitro* method according to Claim 11, wherein the cervical cancer cells are treated with one or more additional chemotherapeutic agents in conjunction with said polypeptide.

14. The *in vitro* method according to Claim 13, wherein the chemotherapeutic agent is selected from the group consisting of bleomycin, chlorambucil, epirubicin, 5-fluorouracil, ifosfamide, mitomycin, methotrexate, vincristine, cisplatin, and vinblastine.

## Patentansprüche

1. Verwendung eines Polypeptids, ausgewählt aus der Gruppe, bestehend aus:
(a) einem Polypeptid, bestehend aus den Aminosäureresten 33-176 von SEQ ID NO: 2;
(b) einem Polypeptid, bestehend aus den Aminosäureresten 25-176 von SEQ ID NO: 2;
(c) einem Polypeptid, bestehend aus den Aminosäureresten 1-176 von SEQ ID NO: 2;
(d) einem Polypeptid, bestehend aus den Aminosäureresten 25-151 von SEQ ID NO: 4; und
(e) einem Polypeptid, bestehend aus den Aminosäureresten 1-151 von SEQ ID NO: 4;
für die Herstellung eines Medikaments für die Behandlung von Gebärmutterhalskrebs.

2. Verwendung gemäß Anspruch 1 zum Hemmen des Wachstums und/oder der Proliferation von Gebärmutterhalskrebszellen.

3. Verwendung gemäß Anspruch 2, wobei das Medikament zur Verabreichung in Verbindung mit Behandlung der Zellen mit Bestrahlung bestimmt ist.

4. Verwendung gemäß Anspruch 2, wobei das Medikament zur Verabreichung in Verbindung mit Behandlung der Zellen mit einem oder mehreren zusätzlichen chemotherapeutischen Mitteln bestimmt ist.

5. Verwendung gemäß Anspruch 4, wobei das chemotherapeutische Mittel aus der Gruppe, bestehend aus Bleomycin, Chlorambucil, Epirubicin, 5-Fluoruracil, Ifosfamid, Mitomycin, Methotrexat, Vincristin, Cisplatin und Vinblastin, ausgewählt ist.

6. Polypeptid, ausgewählt aus der Gruppe, bestehend aus:
(a) einem Polypeptid, bestehend aus den Aminosäureresten 33-176 von SEQ ID NO: 2;
(b) einem Polypeptid, bestehend aus den Aminosäureresten 25-176 von SEQ ID NO: 2;
(c) einem Polypeptid, bestehend aus den Aminosäureresten 1-176 von SEQ ID NO: 2;
(d) einem Polypeptid, bestehend aus den Aminosäureresten 25-151 von SEQ ID NO: 4; und
(e) einem Polypeptid, bestehend aus den Aminosäureresten 1-151 von SEQ ID NO: 4;
zur Verwendung bei der Behandlung von Gebärmutterhalskrebs.

7. Polypeptid zur Verwendung gemäß Anspruch 6 zum Hemmen des Wachstums und/oder der Proliferation von Gebärmutterhalskrebszellen.

8. Polypeptid zur Verwendung gemäß Anspruch 7 zur Verabreichung in Verbindung mit Behandlung der Zellen mit Bestrahlung.

9. Polypeptid zur Verwendung gemäß Anspruch 7 zur Verabreichung in Verbindung mit Behandlung der Zellen mit einem oder mehreren zusätzlichen chemotherapeutischen Mitteln.

10. Polypeptid zur Verwendung gemäß Anspruch 9, wobei das chemotherapeutische Mittel aus der Gruppe, bestehend aus Bleomycin, Chlorambucil, Epirubicin, 5-Fluoruracil, Ifosfamid, Mitomycin, Methotrexat, Vincristin, Cisplatin und Vinblastin, ausgewählt ist.

11. *In-vitro*-Verfahren zum Hemmen des Wachstums und/oder der Proliferation von Gebärmutterhalskrebszellen, umfassend Inkontaktbringen der Zellen mit einem Polypeptid, ausgewählt aus der Gruppe, bestehend aus:
(a) einem Polypeptid, bestehend aus den Aminosäureresten 33-176 von SEQ ID NO: 2;
(b) einem Polypeptid, bestehend aus den Aminosäureresten 25-176 von SEQ ID NO: 2;
(c) einem Polypeptid, bestehend aus den Aminosäureresten 1-176 von SEQ ID NO: 2;
(d) einem Polypeptid, bestehend aus den Aminosäureresten 25-151 von SEQ ID NO: 4; und
(e) einem Polypeptid, bestehend aus den Aminosäureresten 1-151 von SEQ ID NO:4.

12. *In-vitro*-Verfahren gemäß Anspruch 11, wobei die Gebärmutterhalskrebszellen mit Bestrahlung in Verbindung mit dem Polypeptid behandelt werden.

13. *In-vitro*-Verfahren gemäß Anspruch 11, wobei die Gebärmutterhalskrebszellen mit einem oder mehreren zusätzlichen chemotherapeutischen Mitteln in Verbindung mit dem Polypeptid behandelt werden.

14. *In-vitro*-Verfahren gemäß Anspruch 13, wobei das chemotherapeutische Mittel aus der Gruppe, bestehend aus Bleomycin, Chlorambucil, Epirubicin, 5-Fluoruracil, Ifosfamid, Mitomycin, Methotrexat, Vincristin, Cisplatin und Vinblastin, ausgewählt ist.

## Revendications

1. Utilisation d'un polypeptide sélectionné parmi le groupe consistant en:
(a) un polypeptide consistant en les résidus d'acides aminés 33-176 de la SEQ ID NO:2,
(b) un polypeptide consistant en les résidus d'acides aminés 25-176 de la SEQ ID NO:2;
(c) un polypeptide consistant en les résidus d'acides aminés 1-176 de la SEQ ID NO:2;
(d) un polypeptide consistant en les résidus d'acides aminés 25-151 de la SEQ ID NO:4; et
(e) un polypeptide consistant en les résidus d'acides aminés 1-151 de la SEQ ID NO:4;
dans la fabrication d'un médicament pour le traitement du cancer du col de l'utérus.

2. Utilisation selon la revendication 1, pour inhiber la croissance et/ou la prolifération de cellules cancéreuses dans le col de l'utérus.

3. Utilisation selon la revendication 2, dans laquelle le médicament est destiné à être administré conjointement avec le traitement des cellules par radiation.

4. Utilisation selon la revendication 2, dans laquelle le médicament est destiné à être administré conjointement avec le traitement des cellules avec un ou plusieurs agents chimiothérapiques supplémentaires.

5. Utilisation selon la revendication 4, dans laquelle l'agent chimiothérapique est sélectionné parmi le groupe consistant en bléomycine, chlorambucil, épirubicine, 5-fluorouracile, ifosfamide, mitomycine, méthothrexate, vincristine, cisplatine et vinblastine.

6. Polypeptide sélectionné parmi le groupe consistant en:
(a) un polypeptide consistant en les résidus d'acides aminés 33-176 de la SEQ ID NO:2;
(b) un polypeptide consistant en les résidus d'acides aminés 25-176 de la SEQ ID NO:2;
(c) un polypeptide consistant en les résidus d'acides aminés 1-176 de la SEQ ID NO:2;
(d) un polypeptide consistant en les résidus d'acides aminés 25-151 de la SEQ ID NO:4; et
(e) un polypeptide consistant en les résidus d'acides aminés 1-151 de la SEQ ID NO:4;
à utiliser dans le traitement du cancer du col de l'utérus.

7. Polypeptide à utiliser selon la revendication 6, pour inhiber la croissance et/ou la prolifération de cellules cancéreuses dans le col de l'utérus.

8. Polypeptide à utiliser selon la revendication 7, destiné à être administré conjointement avec le traitement des cellules par radiation.

9. Polypeptide à utiliser selon la revendication 7, destiné à être administré conjointement avec le traitement des cellules avec un ou plusieurs agents chimiothérapiques supplémentaires.

10. Polypeptide à utiliser selon la revendication 9, où l'agent chimiothérapique est sélectionné parmi le groupe consistant en bléomycine, chlorambucil, épirubicine, 5-fluorouracile, ifosfamide, mitomycine, méthothrexate, vincristine, cisplatine et vinblastine.

11. *Méthode in vitro* d'inhibition de la croissance et/ou de la prolifération de cellules cancéreuses dans le col de l'utérus, comprenant mettre les cellules en contact avec un polypeptide sélectionné parmi le groupe consistant en:
(a) un polypeptide consistant en les résidus d'acides aminés 33-176 de la SEQ ID NO:2;
(b) un polypeptide consistant en les résidus d'acides aminés 25-176 de la SEQ ID NO:2;
(c) un polypeptide consistant en les résidus d'acides aminés 1-176 de la SEQ ID NO:2;
(d) un polypeptide consistant en les résidus d'acides aminés 25-151 de la SEQ ID NO:4; et
(e) un polypeptide consistant en les résidus d'acides aminés 1-151 de la SEQ ID NO:4.

12. Méthode *in vitro* selon la revendication 11, dans laquelle les cellules cancéreuses dans le col de l'utérus sont traitées par radiation conjointement avec ledit polypeptide.

13. Méthode *in vitro* selon la revendication 11, dans laquelle les cellules cancéreuses dans le col de l'utérus sont traitées avec un ou plusieurs agents chimiothérapiques supplémentaires conjointement avec ledit polypeptide.

14. Méthode *in vitro* selon la revendication 13, dans laquelle l'agent chimiothérapique est sélectionné parmi le groupe consistant en bléomycine, chlorambucil, épirubicine, 5-fluorouracile, ifosfamide, mitomycine, méthothrexate, vincristine, cisplatine et vinblastine.
